**Europäisches Patentamt**

**European Patent Office**

**Office européen des brevets**

(19)

(11) Publication number : **0 500 589 B1**

(12) **EUROPEAN PATENT SPECIFICATION**

(45) Date of publication of patent specification :
16.02.94 Bulletin 94/07

(51) Int. Cl.⁵ : **C07K 13/00, A61K 35/56,
A61K 37/02**

(21) Application number : **90915915.4**

(22) Date of filing : **26.10.90**

(86) International application number :
**PCT/US90/06185**

(87) International publication number :
**WO 91/07435 30.05.91 Gazette 91/12**

(54) **PURIFIED PROTEIN AND BIOACTIVE PHARMACEUTICAL.**

(30) Priority : **13.11.89 US 436141
18.05.90 US 526314**

(43) Date of publication of application :
**02.09.92 Bulletin 92/36**

(45) Publication of the grant of the patent :
**16.02.94 Bulletin 94/07**

(84) Designated Contracting States :
**AT BE CH DE DK ES FR GB GR IT LI LU NL SE**

(56) References cited :
**WO-A-89/09606
Dialog Information Services, File 155, Medline
67-91. NLM accessionnumber 07384445,
Mikulski SM et al: "Tamoxifen and trif-
luouroperazine (Stelazine) potentiate cytos-
tatic/cytotoxic effects of p-30 protein, a novel
protein possessing anti-tumor activity", Cell
Tissue Kinet May 1990, 23(3) p 237-46**

(56) References cited :
**Dialog Information Services, File 55, Biosis
85-91, Biosis number 89101888, Shibuya E K et
al: "Molecular characteristics of cytostatic
factors in amphibian eg cytosols", Develop-
ment (CAMB) 106(4), 1989, 799-808
Dialog Information Services, File 155, Medline
67-91, NLM accession no. 88313378, Shibuya E
K: "Stabilization and enhancement of primary
cytostatic factor (CSF) by ATP and NaF in
amphibian egg cytosols", Dev Biol Sep 1988,
129(1) p 253-64**

(73) Proprietor : **ALFACELL CORPORATION
225 Belleville Avenue
Bloomfield NJ 07003 (US)**

(72) Inventor : **ARDELT, Wojciech, J.
103 Brook Avenue
Passaic, NJ 07055 (US)**
Inventor : **MIKULSKI, Stanislaw, M.
47 Avon Drive
Essex Fells, NJ 07021 (US)**

(74) Representative : **Andrae, Steffen, Dr. et al
Patentanwälte Andrae, Flach, Haug, Kneissl
Balanstrasse 55
D-81541 München (DE)**

EP 0 500 589 B1

## Description

The invention relates to pharmaceuticals, and more particularly relates to pharmaceuticals for use in treating tumors in humans.

At present, tumors are treated either by chemotherapy, radiotherapy or surgery. Each of these therapies has disadvantages.

It would be advantageous to avoid the disadvantages of chemotherapy, radiotherapy and surgery.

One object of the invention is to provide a pharmaceutical useful for the therapy for tumors in humans.

Another object is to provide such pharamceutical which has less disadvantageous side effects than those of other known therapies.

A further object is to provide such a pharmaceutical for use with more than one type of tumor.

Still a further object is, in general, to improve on known therapies for treatment of tumors in humans by providing new pharmaceuticals.

In accordance with EP-A-440 633 (WO 8909606), there is provided a pharmaceutical for treatment of tumors in humans. The pharmaceutical is described as a pure protein having a molecular weight of approximately 12,000 Daltons by mass spectrometry (approximately 14,500 Daltons by electrophoresis), a characteristic isoelectric point by isoelectric focusing and a characteristic amino acid composition. Advantageously although not necessarily, the pharmaceutical is derived from frog eggs subjected to fertilization; in a preferred embodiment, the pharmaceutical is derived from embryos and eggs of the rana pipiens frog. The development of the embryos is advantageously halted before gastrulation and preferably at or before the full blastulae (128 cell) stage, and the embryos are homogenized in the presence of a weakly acidic buffer and then centrifuged to derive a supernatant liquid. This is then subjected to ion-exchange chromatography and size-exclusion chromatography.

It is stated that the pharmaceutical has been characterized to some extent, but the sequence and composition taught in EP-A-440 633 are erroneous in certain respects.

According to the present invention, a pharmaceutical as it can be obtained in accordance with EP-A-440 633, is further characterized as being a pure protein having the amino acid sequence set forth in claim 9.

2 The present invention further comprises a pharmaceutical comprising a combination of the product described in EP-A-440 633 with one of the further drugs having the International Non-Proprietary Names (INN) tamoxifen and trifluoperazine respectively.

When such combined pharmaceutical is used in vitro, the results of the combined therapy are, in certain instances, much more bioactive than would be expected.

## BRIEF DESCRIPTION OF THE DRAWINGS

The invention will be better understood with reference to the following illustrative and non-limiting drawings, in which:

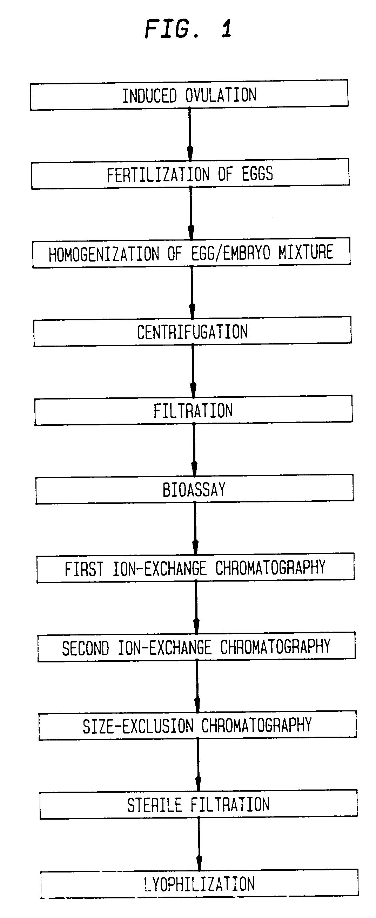

Figure 1 is a flow chart of the process in accordance with a preferred embodiment of the invention; and

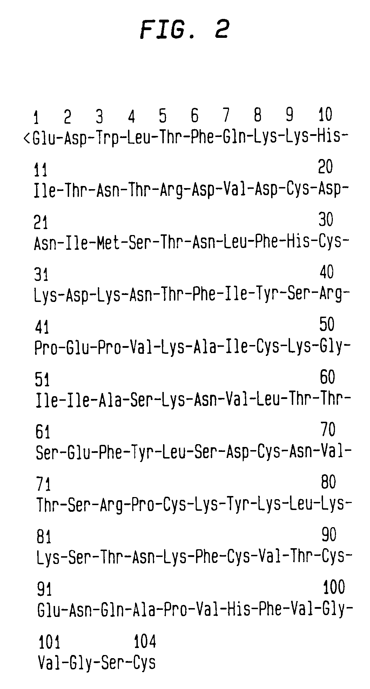

Figure 2 is an illustration of the sequence of amino acids in the pharmaceutical.

Figure 3 shows experimental data illustrating how a combined therapy of the pharmaceutical and STELAZINE has a much greater bioactivity against A-549 human lung carcinoma cells than do either the pharmaceutical or STELAZINE separately.

Figure 4 shows experimental data illustrating how a combined therapy of the pharmaceutical and TAMOXIFEN has a much greater bioactivity against ASPC-1 human pancreatic adenocarcinoma cells than do either the pharmaceutical or TAMOXIFEN separately.

## DETAILED DESCRIPTION OF PREFERRED EMBODIMENTS

### A. Production of Embryo/Egg Mixture

In the preferred embodiment, rana pipiens eggs are produced by induced ovulation (so that their development takes place in a highly controlled manner) and fertilized under controlled conditions outside the body of the female frog. Ovulation is only induced during the months from September through March; during the other months, induced ovulation is not feasible. This is because rana pipiens ovulates spontaneously in the month of April and its breeding season lasts from May through August.

Only large, healthy and vigorous gravid female rana pipiens are selected for induced ovulation. They are separated from male rana pipiens and are maintained at a temperature of 6°C for a period of three days in tanks filled with one inch of tap water. This temperature is preferred, but other temperatures can be used if the other variables in the fertilization process are accordingly adjusted; the rate at which the eggs develop is dependent upon temperature.

For each selected female, a petri dish is preferably filled with 10 cubic centimeters of tested spring water. Tested spring water is fresh spring water which has been tested to support life of rana pipiens and its embryos.

Advantageously, each selected female is induced to ovulate by introducing 5 freshly isolated female rana pipiens pituitary glands into her body. Male pituitary glands may be substituted for female pituitary glands, but one female pituitary gland is equivalent to two male pituitary glands and the quantities used must be adjusted accordingly.

The appropriate number of glands is placed in the corresponding liquid-containing petri dish. Each selected female is brought to room temperature (22°C). Dish by dish, the glands are drawn up into a syringe and introduced into the right lower quadrant of the abdomen of the corresponding selected female by injection through an 18 gauge needle.

The selected females are then replaced in tanks filled with one inch of spring water. Flat rocks are placed in the bottom of each tank, so that the females can rest upon them and remain above the water line. (This is advantageous because gravid females can become lethargic and may drown if not held above the water line.) The tanks are covered with warehouse cloth (to prevent the frogs from jumping out of the tanks) and advantageously kept at room temperature (22°C) for 48 hours. The eggs produced by the gravid females are then, in accordance with the preferred embodiment, subjected to fertilization outside the bodies of the female frogs, advantageously in petri dishes, and preferably in 4 petri dishes per gravid female.

To accomplish this fertilization, male rana pipiens are sacrificed (as by over-etherization) and their testes are removed and cleaned of connective tissue and fat. Enough males must be killed to yield at least 4 testes per petri dish of eggs to be fertilized (i.e. 16 testes per gravid female). This quantity produces an optimized quantity of sperm suspension considering the size of the petri dishes. (Advantageously, the pituitary glands of the male rana pipiens are also removed for subsequently inducing ovulation in other females.)

Four testes are then placed in each petri dish, and the testes are macerated (as by chopping) to form a milky sperm suspension. The maceration must be conducted in such a manner as not to chop the sperm. The eggs are then removed from each gravid female by pressing her abdomen towards her posterior. The egg production of each female is distributed evenly among four suspension-filled petri dishes; this avoids overcrowding the eggs in the dishes.

The eggs are left in the suspension for about 3 to 4.5 hours at room temperature. During the first hour, the sperm suspension and eggs in the dish are intermittently swirled so that the eggs are always covered by the sperm suspension. After the 3 to 4.5 hours have passed, the dishes are checked under a dissecting microscope for signs of cleavage. When 80% cleavage of the rana pipiens embryos is observed, the corresponding dish is collected; the embryos are then in at least the 4 cell stage of development. The 4 cell stage of development is used as a benchmark because it establishes division of the eggs and the existing fact of fertilization cannot be overlooked.

Since 100% cleavage of the embryos is not ordinarily achieved in the stated 3 to 4.5 hour time, the collected dishes will ordinarily contain both embryos (fertilized eggs) and unfertilized eggs. This mixture will be occasionally referred to as a mixture of eggs subjected to fertilization, meaning that both eggs and embryos are present. Since 80% cleavage is used as a benchmark in the preferred embodiment, the ratio of eggs to embryos in the mixture is approximately 1:4.

All collected eggs subjected to fertilization may then be scraped into containers and stored in frozen form at -15°C to -20°C. This storage is not essential for the practice of the invention; it is preferred only when it is convenient to carry out subsequent processing in batches.

B. Mechanical Processing of the Eggs Subjected to Fertilization

If the mixture has been frozen, it is thawed by any method which does not overheat it. The thawed or never-frozen mixture is then homogenized in the presence of a weakly acidic buffer, preferably under a laminar air flow hood to avoid contamination.

In the preferred embodiment, the mixture of eggs subjected to fertilization is mixed, at room temperature, with 0.15M sodium acetate (pH 4.8 - 4.9) using two volumes of buffer for one like volume of the mixture of eggs subjected to fertilization. The buffer need not be sodium acetate but must be weakly acidic; sodium acetate is used because, in the preferred embodiment, S-Sepharose chromatography is carried out and sodium acetate is a good buffer within a pH range of 4 - 5.8 (in which range S-Sepharose exchange columns are efficient). Homogenization is carried out in a Waring Blender until all eggs have been disrupted as observed visually, but a Waring Blender is not required and any sanitary method for accomplishing thorough homogenization can be used. Homogenization is complete when the suspension appears homogenous with no visual sign of intact eggs. The homogenate is then stirred at room temperature for 2 hours, and kept frozen until further processing is to be carried out.

In the preferred embodiment, the stirred.homogenized mixture of eggs subjected to fertilization (which mixture was kept frozen for at least a week and then thawed) is centrifuged (at 4°C to 8°C) in two stages. In the first stage, the stirred homogenate is centrifuged at an average acceleration of 34,000 x g and the resulting supernatant is saved. The time required for this step is usually 60 minutes, but it is necessary to obtain clear and gel-free supernatant and the time is increased as necessary to achieve this. In the second stage, the sediment pellet which results from the first stage of centrifugation is re-homogenized as above. The re-homogenized sediment is then centrifuged as above, and the resulting supernatant is then pooled with the supernatant produced in the first step.

The duration, speed, and other particulars of the centrifugation steps described above are not required to practice the invention, but they are optimized for the preferred embodiment of the invention.

As each batch of supernatant fluid is decanted, it is filtered through a layer of DEAE-Sepharose equilibrated in the extraction buffer (0.15M sodium acetate, pH 4.8 - 4.9). This is to remove debris which could clog the columns in the processing steps described below, but the use of DEAE-Sepharose is not required and other suitable filters could be used instead.

In the preferred embodiment, the filtered extract is assayed for bioactivity against a predetermined cell line. This is not required but, for two main reasons, is advantageous. The first reason is that in a process which is scaled to commercial production quantities, batches of filtered extract are pooled together before subsequent processing steps are undertaken. By checking for and discarding batches of inactive filtered extract, inadvertent contamination of bioactive batches with nonbioactive ones is eliminated. The other reason is that the subsequent processing steps are expensive, and identification and rejection of nonbioactive material saves the substantial expense which would otherwise be wasted on processing it. However, even though an assay is presently preferred, no assayed batch of filtered extract has ever been found to be inactive and the use of an assay must therefore be considered entirely optional.

The actual assay used in the preferred embodiment is performed using human submaxillary epidermoid carcinoma A-253 cells and a tetrazolium compound sold by Chemicon International, Inc., 100 Lomita Street, El Segundo, California, under the MTT trademark. However, this is not necessary and other bioassays may be used. Alternatively, a bioassay may be unnecessary if an alternate non-bioassay method which correlates well with a bioassay is available.

The filtered extract is advantageously immediately subjected to further purification steps.

## C. Ion-Exchange Chromatography

The filtered extract is subjected to ion-exchange chromatography. In the preferred embodiment, the ion-exchange chromatography is carried out in two steps under slightly different conditions. During the first step, the active protein is initially isolated and essentially freed of endotoxin. During the second step, the protein is purified from other proteins which have been co-purified with the active protein during the first step as well as from any possible persisting endotoxin.

In the preferred embodiment, the first ion-exchange chromatography step is carried out using a column which is 11 cm in diameter and 20 cm long. The conditions described below are optimized for columns of these dimensions. However, if differently-dimensioned columns are used, the conditions may change.

In the preferred embodiment, the purpose of the two consecutive ion-exchange chromatography steps is to isolate proteins with isoelectric points pI of 9.5 - 10.5 (as determined by isoelectric focusing) before separating the proteins by size. In the first ion-exchange chromatography step, the pH of the filtered extract is adjusted to 5.2 with ammonium hydroxide and loaded onto a column which is filled with S-Sepharose. The column is equilibrated in a 0.15M sodium acetate buffer (pH 5.2) and the column is developed with a continuous linear gradient of sodium chloride (0 - 0.5M) made in the equilibrating buffer. These conditions are not necessary to practice the invention but they are convenient and, at present, seem to produce good working yields of bioactive protein.

In the preferred embodiment, the eluted protein is then diluted 2 times with pyrogen-free water and subjected to a second ion-exchange chromatography step which is carried out under different conditions. This second ionexchange chromatography step is performed on a second column which is 11 cm in diameter and 15 cm long and is filled with S-Sepharose. The column is equilibrated in a 0.15M sodium acetate buffer (pH 5.2) and the column is developed with a continuous linear gradient of sodium chloride (0 - 0.3M) made in the equilibrating buffer.

Advantageously, both chromatography steps are carried out at 18°C - 20°C (air conditioned room), but this is not critical. Column chromatography is known to be more efficient above 4°C (cold-room) temperatures and the process is carried out at the highest temperature which is consistent with stability of the purified pharmaceutical.

In the preferred embodiment, the eluate from the second ion-exchange step is alkalized to pH 6 - 7 with ammonium hydroxide, concentrated by ultrafiltration using a membrane which has a 5000 Daltons molecular weight cutoff and discarding the permeate. Suitable membranes are the Spectra-Por (manufactured by Spectrum Medical Industries) and the Amicon YM5 (manufactured by Amicon), but other membranes and other concentration procedures may be used instead.

## D. Size-Exclusion Chromatography

The concentrated material is then loaded onto a column which is 11 cm in diameter and 50 cm long, which is filled with Bio-Gel P-30 gel and which is equilibrated in 0.075 ammonium bicarbonate. The main protein peak is isolated.

These specific conditions are not required to practice the invention; other dimensions, gels and even other size-exclusion techniques could be used instead. However, it is recommended that the size-exclusion chromatography follow the ion-exchange chromatography. This is because this order of chromatography makes it possible to use a column of reasonable size for the size-exclusion chromatography.

## E. Final Processing

The eluate from the size-exclusion column is then sterile filtered through a 0.22 micron filter and subsequently lyophilized (freeze-dried). These process steps are standard in the pharmaceutical industry, and are not a part of the invention. The resultant preparation is devoid of viable micro-organisms.

## Bioactivity of the Pharmaceutical

Confirmatory in vitro and in vivo animal data show that the pharmaceutical is active against human submaxillary epidermoid carcinoma A-253 cells and human ovarian adenocarcinoma NIH-OVCAR-3 cells. The pharmaceutical has also shown activity against human leukemic HL-60 cells, human COLO 320 DM cells originally isolated from colon adenocarcinoma, human LOX melanoma, and human lung squamous carcinoma HT-520 cells.

In vitro data indicate that a combination of the pharmaceutical with a drug sold under the TAMOXIFEN trademark is much more bioactive against human pancreatic ASPC-1 adenocarcinoma than would be expected, given the separate activities of the pharmaceutical and TAMOXIFEN. In vitro data also indicate that a combination of the pharmaceutical with a drug sold under the STELAZINE trademark is much more bioactive against human lung A-549 carcinoma than would be expected, given the separate activities of the pharmaceutical and STELAZINE.

The preferred embodiment of the invention was tested using a cell culture assay. In such an assay, a cell line of known growth rate over a predetermined period is treated with the substance under test and the growth of the treated cells is compared with the growth which would ordinarily be expected from untreated cells.

The pharmaceutical was dissolved in phosphate buffered saline (PBS) to obtain 1mg/ml stock solution.

TAMOXIFEN is produced by Sigma Chemical Co., St. Louis MO and is an INN for (Z-1-p-dimethylaminoethoxyphenyl-1,2-diphenyl-1-butene), citrate salt. In the experiments described herein, TAMOXIFEN was dissolved in absolute ethanol and diluted with RPMI 1640 medium (as produced by Hazleton Research Products, Lenexa, KS) to obtain 1mM stock solution (final concentration of ethanol 11%).

STELAZINE is produced by SK&F Co., subsidiary of SmithKline Beckman Co., Carolina P.R. and is a trademark for (10-[3-(4-methylpiperazin-1-yl)-propyl]-2-trifluoromethylphenothiazine) (INN: trifluoperazine). In the experiments described herein, STELAZINE was diluted with RPMI 1640 medium to obtain 1mM stock solution.

The assay system utilized the ASPC-1 human pancreatic adenocarcinoma cell line and the A-549 human lung carcinoma cell line; both lines were obtained from the American Type Culture Collection (accession numbers were ATCC CRL 1682 for ASPC-1 and ATCC CCL 185 for A-549). Both cell lines were cultured in RPMI 1640 medium (Hazleton Research Products) and supplemented with 20% (ASPC-1) or 10% (A-549) heat-inactivated fetal bovine serum (Gibco Life Technologies, Grand Island NY) and antibiotic-antimycotic solution composed of: 10,000 units per 1 ml penicillin, 10 mg per 1 ml streptomycin and 25 µg per 1 ml fungizone (complete growth medium). The cells were seeded into 96-well tissue culture plates manufactured by Falcon, of Oxnard, CA at a density of 2000 viable cells (50 µl per well) for the A-549 cell line and 4000 viable cells (50 µl) per well for the ASPC-1 cell line. The cell number was based on the previously determined growth curve characteristics for seven days of culture. The cells were allowed to settle for 24 hours and then 50 µl of the phar-

5

maceutical and/or TAMOXIFEN or STELAZINE solutions were added per well. The following final concentrations were used:

a) the pharmaceutical, 20ng to 10 µg/ml;

b) TAMOXIFEN, 10 µM for ASPC-1 cells; and

c) STELAZINE, 5 µM.

The plates were incubated for an additional six days at 37 °C and 5% carbon dioxide atmosphere. The total assay time was consequently seven days (one day in which the cells are allowed to settle, and six days of incubation). Percentages of viable cells were then determined using the MTT colorimetric assay using the Bio-Rad EIA microtiter plate reader.

The number of cells was determined by a direct count in an AO-Spencer "Brightline" hemocytometer manufactured by Reichert Scientific Instruments in Buffalo, NY with a Neubauer ruling. All solutions used for this purpose were manufactured by Hazleton Research Products. Attached cells were washed three times with Hanks' Balanced Salt Solution and treated with 2 ml of a 0.25% Trypsin - 0.02% EDTA solution in buffered saline for about thirty seconds. The solution was removed and the cells were left at 37 °C for 10 minutes, then suspended in 10 ml of the complete growth medium. The 0.25 ml of the cell suspension was diluted with 0.75 ml of the complete growth medium and then 1 ml of 0.5% Trypan Blue solution was added and viable cells were counted.

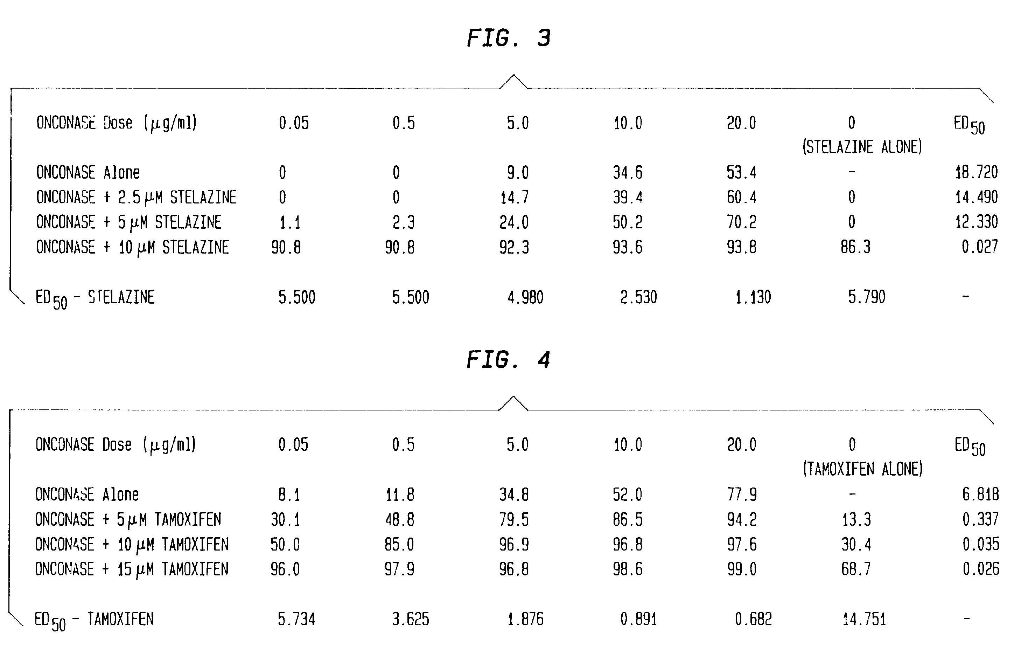

Figures 3 and 4 present the result of the above experiments. These tables are expressed in mean percent of growth inhibition, i.e. they indicate the effectiveness with which the tested therapies prevented the tested cancer cells from growing over the one week duration of the assay. Thus, a higher number indicates a higher bioactivity against the cell line used in the experiment.

These results demonstrate that, in the instances shown, the bioactivities of the pharmaceutical combined with STELAZINE in the case of A-549 human lung carcinoma and the pharmaceutical combined with TAMOXIFEN in the case of ASPC-1 human pancreatic adenocarcinoma are much greater than would be expected from the bioactivities of the individual drugs alone. This may be seen from the $ED_{50}$ figures which are along the right edge of the Tables. These figures represent computed isoeffective doses; the figure shown is the amount of material which would be required to halve the growth rate of the cells undergoing the assay. Thus, the lower the $ED_{50}$ figure, the smaller the dose required to achieve the same bioactivity.

## Chemical Analysis and Composition of the Pharmaceutical

The pharmaceutical described above has been well characterized chemically. While the pharmaceutical is a protein isolated from rana pipiens, it is believed that the pharmaceutical may be produced using genetic engineering techniques, as long as the end result has the following chemistry and structure:

The pharmaceutical is a pure protein (i.e. homogeneous, as established by standard tests which are used to assay the homogeneity of proteins). By electrophoresis, the molecular weight of the pharmaceutical is approximately 14,500 Daltons. Calculation of the molecular weight based upon the below listed amino acid sequence indicates that the molecular weight should be 11,819 Daltons. However, because metal ions may have bonded to the protein despite all efforts to remove them, and because different isotopes may be involved, the molecular weight of the pharmaceutical as determined by mass spectroscopy is 12,430 Daltons. In view of this discrepancy, the molecular weight of the pharmaceutical as determined by mass spectrometry will be considered to be approximately 12,000 Daltons. The pharmaceutical has an isoelectric point pI between 9.5 and 10.5, as determined by isoelectric focusing. The pharmaceutical has a blocked amino terminal group and is essentially free of carbohydrates (as determined by anthrone and orcinol methods).

The pharmaceutical has the following amino acid composition:

Amino Acid Analysis

| AMINO ACID RESIDUE | MOL %<br>(24 HOUR ACID HYDROLYSIS) | |
|---|---|---|
| Aspartic acid/Asparagine | 13.99 | |
| Threonine | 9.30 | (Note 1) |
| Serine | 7.78 | |
| Glutamic acid/Glutamine | 6.10 | |
| Proline | 4.36 | |
| Glycine | 3.09 | |
| Alanine | 3.09 | |
| Cystine/2 | 6.92 | (Note 1) |
| Valine | 8.20 | |
| Methionine | 0.85 | (Note 1) |
| Isoleucine | 4.86 | (Note 2) |
| Leucine | 5.22 | |
| Tyrosine | 2.96 | |
| Phenylalanine | 6.05 | |
| Histidine | 2.88 | |
| Lysine | 11.62 | |
| Arginine | 2.70 | |
| Tryptophan | Not Determined | (Note 3) |
| Approximate Total | 99.97 % | |

Note 1: Threonine, cystine/2 and methionine are partially destroyed during hydrolysis and this value is uncorrected for such partial destruction.

Note 2: This value is uncorrected for incomplete hydrolysis.

Note 3: Tryptophan cannot be detected in acid hydrolysis of proteins because it is destroyed and is consequently shown as Not Determined. However, analysis of the ultraviolet spectrum revealed the presence of one tryptophan residue per molecule.

```
                        Amino  Acid  Composition
                (as  calculated  from  amino  acid  sequence)

        AMINO ACID                              NUMBER OF RESIDUES
                                                    PER MOLECULE


        Aspartic acid                                   6
        Asparagine                                      8
        Threonine                                      10
        Serine                                          8
        Glutamic acid                                   3
        Pyroglutamic acid                               1
        Glutamine                                       2
        Proline                                         4
        Glycine                                         3
        Alanine                                         3
        Cystine/2                                       8
        Valine                                          8
        Methionine                                      1
        Isoleucine                                      6
        Leucine                                         5
        Tyrosine                                        3
        Phenylalanine                                   6
        Histidine                                       3
        Lysine                                         12
        Arginine                                        3
        Tryptophan                                      1


        Approximate Total                             104
```

The pharmaceutical has been sequenced. As is shown below, the total length of the sequence is believed to be 104 residues. The N-terminus of the protein is pyroglutamic acid (<Glu). This is a cyclized derivative of glutamic acid which is devoid of the free amino group necessary for direct sequencing and which therefore "blocks" the N-terminus of the protein.

When the shorter fragment described in EP-A-440 633 was cleaved with pyroglutamate aminopeptidase, pyroglutamic acid was removed from the shorter fragment, permitting sequencing to commence at the second residue. Such cleavage is a strong indication that the N-terminus is pyroglutamic acid since pyroglutamate aminopeptidase only cleaves pyroglutamic acid. The presence of pyroglutamic acid was further confirmed by mass spectrometry of the referenced shorter fragment. The molecular weight of this shorter fragment determined by mass spectrometry agreed well with the weight as calculated assuming that pyroglutamic acid was present and disagreed with the weight as calculated assuming that glutamic acid was present.

The pharmaceutical has the following amino acid sequence:

```
     1    2    3    4    5    6    7    8    9   10
   <Glu-Asp-Trp-Leu-Thr-Phe-Gln-Lys-Lys-His-

    11                                       20
   Ile-Thr-Asn-Thr-Arg-Asp-Val-Asp-Cys-Asp-

    21                                       30
   Asn-Ile-Met-Ser-Thr-Asn-Leu-Phe-His-Cys-

    31                                       40
   Lys-Asp-Lys-Asn-Thr-Phe-Ile-Tyr-Ser-Arg-

    41                                       50
   Pro-Glu-Pro-Val-Lys-Ala-Ile-Cys-Lys-Gly-

    51                                       60
   Ile-Ile-Ala-Ser-Lys-Asn-Val-Leu-Thr-Thr-

    61                                       70
   Ser-Glu-Phe-Tyr-Leu-Ser-Asp-Cys-Asn-Val-

    71                                       80
   Thr-Ser-Arg-Pro-Cys-Lys-Tyr-Lys-Leu-Lys-

    81                                       90
   Lys-Ser-Thr-Asn-Lys-Phe-Cys-Val-Thr-Cys-

    91                                      100
   Glu-Asn-Gln-Ala-Pro-Val-His-Phe-Val-Gly-

   101            104
   Val-Gly-Ser-Cys
```

The C-terminus at position 104 has been identified to be cysteine.

Although a preferred embodiment has been described above, the scope of the invention is limited only by the following claims:

## Claims

1. A bioactive pharmaceutical comprising

a protein having a molecular weight of approximately 12,000 Daltons by mass spectrometry or approximately 14,500 Daltons by gel electrophoresis, and an amino acid composition approximately as follows:

| AMINO ACID RESIDUE | MOL % (24 HOUR ACID HYDROLYSIS) |
|---|---|
| Aspartic acid/Asparagine | 13.39 |
| Threonine | 9.84 |
| Serine | 8.08 |
| Glutamic acid/Glutamine | 5.88 |
| Proline | 3.98 |
| Glycine | 2.98 |
| Alanine | 2.92 |
| Cystine/2 | 7.77 |
| Valine | 7.77 |
| Methionine | 0.94 |
| Isoleucine | 5.29 |
| Leucine | 4.95 |
| Tyrosine | 2.85 |
| Phenylalanine | 5.73 |
| Histidine | 2.99 |
| Lysine | 11.78 |
| Arginine | 2.85 |
| Tryptophan | Not Determined |
| Approximate Total | 99.99 % |

and either (Z)-1-p-dimethylaminoethoxyphenyl-1,2-diphenyl-1-butene, citrate salt (tamoxifen, citrate salt) or 10-[3-(4-methylpiperazin-1-yl)-propyl]-2-trifluoromethylphenothiazine (trifluoperazine).

2. A bioactive pharmaceutical comprising
    a protein having a molecular weight of approximately 12,000 Daltons by mass spectrometry or approximately 14,500 Daltons by gel electrophoresis, and an amino acid composition approximately as follows:

| AMINO ACID | NUMBER OF RESIDUES PER MOLECULE |
|---|---|
| Aspartic acid | 6 |
| Asparagine | 8 |
| Threonine | 10 |
| Serine | 8 |
| Glutamic acid | 3 |
| Pyroglutamic acid | 1 |
| Glutamine | 2 |
| Proline | 4 |
| Glycine | 3 |
| Alanine | 3 |
| Cystine/2 | 8 |
| Valine | 8 |
| Methionine | 1 |
| Isoleucine | 6 |
| Leucine | 5 |
| Tyrosine | 3 |
| Phenylalanine | 6 |
| Histidine | 3 |
| Lysine | 12 |
| Arginine | 3 |
| Tryptophan | 1 |
| Approximate Total | 104 |

and either (Z)-1-p-dimethylaminoethoxyphenyl-1,2-diphenyl-1-butene, citrate salt or 10-[3-(4-methylpiperazin-1-yl)-propyl)-2-trifluoromethylphenothiazine.

3. A bioactive pharmaceutical comprising

a pure protein obtainable from frog eggs which have been subjected to fertilization, the protein having a molecular weight of approximately 12,000 Daltons by mass spectrometry, an isoelectric point pI of 9.5 to 10.5 by isoelectric focusing and a blocked amino terminal group, the protein being essentially free of carbohydrates, and

either (Z)-1-p-dimethylaminoethoxyphenyl-1,2-diphenyl-1-butene, citrate salt or 10-[3-(4-methylpiperazin-1-yl)-propyl]-2-trifluoromethylphenothiazine.

4. The combination of claim 3, wherein the predominant amino acids are lysine and threonine.

5. The combination of claim 3, wherein each molecule of the protein contains exactly one residue of tryptophan.

6. The combination of claim 3, wherein each molecule of the protein contains exactly one residue of methionine.

7. A bioactive pharmaceutical comprising

a protein having an amino acid sequence which is at least 60% homologous to the following amino acid sequence:

11

```
 1    2    3    4    5    6    7    8    9    10
<Glu-Asp-Trp-Leu-Thr-Phe-Gln-Lys-Lys-His-

11                                        20
Ile-Thr-Asn-Thr-Arg-Asp-Val-Asp-Cys-Asp-

21                                        30
Asn-Ile-Met-Ser-Thr-Asn-Leu-Phe-His-Cys-

31                                        40
Lys-Asp-Lys-Asn-Thr-Phe-Ile-Tyr-Ser-Arg-

41                                        50
Pro-Glu-Pro-Val-Lys-Ala-Ile-Cys-Lys-Gly-

51                                        60
Ile-Ile-Ala-Ser-Lys-Asn-Val-Leu-Thr-Thr-

61                                        70
Ser-Glu-Phe-Tyr-Leu-Ser-Asp-Cys-Asn-Val-

71                                        80
Thr-Ser-Arg-Pro-Cys-Lys-Tyr-Lys-Leu-Lys-

81                                        90
Lys-Ser-Thr-Asn-Lys-Phe-Cys-Val-Thr-Cys-

91                                        100
Glu-Asn-Gln-Ala-Pro-Val-His-Phe-Val-Gly-

101            104
Val-Gly-Ser-Cys
```

and either (Z)-1-p-dimethylaminoethoxyphenyl-1,2-diphenyl-1-butene, citrate salt or 10-[3-(4-methylpiperazin-1-yl)-propyl]-2-trifluoromethylphenothiazine.

8. A bioactive pharmaceutical comprising
    a protein having an amino acid fragment which is at least 15 amino acid residues long and which fragment has an amino acid sequence that matches any equally long fragment of the following amino acid sequence:

```
   1    2    3    4    5    6    7    8    9    10
<Glu-Asp-Trp-Leu-Thr-Phe-Gln-Lys-Lys-His-

  11                                        20
Ile-Thr-Asn-Thr-Arg-Asp-Val-Asp-Cys-Asp-

  21                                        30
Asn-Ile-Met-Ser-Thr-Asn-Leu-Phe-His-Cys-

  31                                        40
Lys-Asp-Lys-Asn-Thr-Phe-Ile-Tyr-Ser-Arg-

  41                                        50
Pro-Glu-Pro-Val-Lys-Ala-Ile-Cys-Lys-Gly-

  51                                        60
Ile-Ile-Ala-Ser-Lys-Asn-Val-Leu-Thr-Thr-

  61                                        70
Ser-Glu-Phe-Tyr-Leu-Ser-Asp-Cys-Asn-Val-

  71                                        80
Thr-Ser-Arg-Pro-Cys-Lys-Tyr-Lys-Leu-Lys-

  81                                        90
Lys-Ser-Thr-Asn-Lys-Phe-Cys-Val-Thr-Cys-

  91                                       100
Glu-Asn-Gln-Ala-Pro-Val-His-Phe-Val-Gly-

  101           104
Val-Gly-Ser-Cys
```

and either (Z)-1-p-dimethylaminoethoxyphenyl-1,2-diphenyl-1-butene, citrate salt or 10-[3-(4-methylpiperazin-1-yl)-propyl]-2-trifluoromethylphenothiazine.

9.   A protein having the following amino acid sequence:

```
 1    2    3    4    5    6    7    8    9    10
<Glu-Asp-Trp-Leu-Thr-Phe-Gln-Lys-Lys-His-

11                                       20
Ile-Thr-Asn-Thr-Arg-Asp-Val-Asp-Cys-Asp-

21                                       30
Asn-Ile-Met-Ser-Thr-Asn-Leu-Phe-His-Cys-

31                                       40
Lys-Asp-Lys-Asn-Thr-Phe-Ile-Tyr-Ser-Arg-

41                                       50
Pro-Glu-Pro-Val-Lys-Ala-Ile-Cys-Lys-Gly-

51                                       60
Ile-Ile-Ala-Ser-Lys-Asn-Val-Leu-Thr-Thr-

61                                       70
Ser-Glu-Phe-Tyr-Leu-Ser-Asp-Cys-Asn-Val-

71                                       80
Thr-Ser-Arg-Pro-Cys-Lys-Tyr-Lys-Leu-Lys-

81                                       90
Lys-Ser-Thr-Asn-Lys-Phe-Cys-Val-Thr-Cys-

91                                       100
Glu-Asn-Gln-Ala-Pro-Val-His-Phe-Val-Gly-

101         104
Val-Gly-Ser-Cys
```

## Patentansprüche

1. Bioaktives Pharmazeutikum mit
   einem Protein mit einem Molekulargewicht von etwa 12.000 Dalton, bestimmt durch Massenspektrometrie, oder etwa 14,500 Dalton, bestimmt durch Gel-Elektrophorese, sowie einer Aminosäurezusammensetzung, die etwa wie folgt ist:

| Aminosäure | MOL % (24 stündige saure Hydrolyse) |
|------------|-------------------------------------|
| Asparaginsäure /Asparagin | 13,39 |
| Threonin | 9,84 |
| Serin | 8,08 |
| Glutaminsäure/Glutamin | 5,88 |
| Prolin | 3,98 |
| Glycin | 2,98 |
| Alanin | 2,92 |
| Cystin/2 | 7,77 |
| Valin | 7,77 |
| Methionin | 0,94 |
| Isoleucin | 5,29 |
| Leucin | 4,95 |
| Tyrosin | 2,85 |
| Phenylalanin | 5,73 |
| Histidin | 2,99 |
| Lysin | 11,78 |
| Arginin | 2,85 |
| Tryptophan | nicht bestimmt |
| Angenäherte Gesamtmenge | 99,99 % |

sowie entweder (Z)-1-p-Dimethylaminoethoxyphenyl-1,2-diphenyl-1-buten, Citratsalz (Tamoxifen, Citratsalz), oder 10-[3-(4-Methylpiperazin-1-yl)-propyl]-2-trifluormethylphenothiazin (Trifluoperazin).

2. Bioaktives Pharmazeutikum mit
einem Protein mit einem Molekulargewicht von etwa 12.000 Dalton, bestimmt durch Massenspektrometrie, oder etwa 14.500 Dalton, bestimmt durch Gel-Elektrophorese, und einer Aminosäurezusammensetzung etwa wie folgt:

| Aminosäure | Anzahl der Reste pro Molekül |
|---|---|
| Asparaginsäure | 6 |
| Asparagin | 8 |
| Threonin | 10 |
| Serin | 8 |
| Glutaminsäure | 3 |
| Pyroglutaminsäure | 1 |
| Glutamin | 2 |
| Prolin | 4 |
| Glycin | 3 |
| Alanin | 3 |
| Cystin/2 | 8 |
| Valin | 8 |
| Methionin | 1 |
| Isoleucin | 6 |
| Leucin | 5 |
| Tyrosin | 3 |
| Phenylalanin | 6 |
| Histidin | 3 |
| Lysin | 12 |
| Arginin | 3 |
| Tryptophan | 1 |
| Angenäherter Gesamtwert | 104 |

sowie entweder (Z)-1-p-Dimethylaminoethoxyphenyl-1,2-diphenyl-1-buten, Citratsalz, oder 10-[3-(4-Methylpiperazin-1-yl)-propyl]-2-trifluormethylphenothiazin.

3. Bioaktives Pharmazeutikum mit
einem reinen Protein, das aus befruchteten Froscheiern erhältlich ist, wobei das Protein ein Molekulargewicht von etwa 12.000 Dalton, bestimmt durch Massenspektrometrie, einen isoelektrischen Punkt pI von 9,5 bis 10,5, bestimmt durch isoelektrische Fokussierung, sowie eine blockierte terminale Aminogruppe aufweist, wobei das Protein im wesentlichen frei von Kohlenhydraten ist, sowie
entweder (Z)-1-p-Dimethylaminoethoxyphenyl-1,2-diphenyl-1-buten, Citratsalz, oder 10-[3-(4-Methylpiperazin-1-yl)-propyl]-2-trifluormethylphenothiazin.

4. Kombination nach Anspruch 3, bei der die dominierenden Aminosäuren Lysin und Threonin sind.

5. Kombination nach Anspruch 3, bei der jedes Molekül des Proteins genau einen Tryptophanrest enthält.

6. Kombination nach Anspruch 3, bei der jedes Molekül des Proteins genau einen Methioninrest enthält.

7. Bioaktives Pharmazeutikum, mit
einem Protein einer Aminosäuresequenz, die zu wenigstens 60 % der folgenden Aminosäuresequenz homolog ist:

```
      1    2    3    4    5    6    7    8    9   10
   <Glu-Asp-Trp-Leu-Thr-Phe-Gln-Lys-Lys-His-

     11                                      20
   Ile-Thr-Asn-Thr-Arg-Asp-Val-Asp-Cys-Asp-

     21                                      30
   Asn-Ile-Met-Ser-Thr-Asn-Leu-Phe-His-Cys-

     31                                      40
   Lys-Asp-Lys-Asn-Thr-Phe-Ile-Tyr-Ser-Arg-

     41                                      50
   Pro-Glu-Pro-Val-Lys-Ala-Ile-Cys-Lys-Gly-

     51                                      60
   Ile-Ile-Ala-Ser-Lys-Asn-Val-Leu-Thr-Thr-

     61                                      70
   Ser-Glu-Phe-Tyr-Leu-Ser-Asp-Cys-Asn-Val-

     71                                      80
   Thr-Ser-Arg-Pro-Cys-Lys-Tyr-Lys-Leu-Lys-

     81                                      90
   Lys-Ser-Thr-Asn-Lys-Phe-Cys-Val-Thr-Cys-

     91                                     100
   Glu-Asn-Gln-Ala-Pro-Val-His-Phe-Val-Gly-

     101            104
   Val-Gly-Ser-Cys
```

und entweder (Z)-1-p-Dimethylaminoethoxyphenyl-1,2-diphenyl-1-buten, Citratsalz, oder 10-[3-(4-Methylpiperazin-1-yl)-propyl]-2-trifluormethylphenothiazin.

8.  Bioaktives Pharmazeutikum mit
    einem Protein mit einem Aminosäurefragment, das wenigstens 15 Aminosäurereste lang ist und das eine Aminosäuresequenz aufweist, die irgendeinem gleich langen Fragment der folgenden Aminosäuresequenz entspricht:

```
   1    2    3    4    5    6    7    8    9   10
  Glu-Asp-Trp-Leu-Thr-Phe-Gln-Lys-Lys-His-

  11                                        20
  Ile-Thr-Asn-Thr-Arg-Asp-Val-Asp-Cys-Asp-

  21                                        30
  Asn-Ile-Met-Ser-Thr-Asn-Leu-Phe-His-Cys-

  31                                        40
  Lys-Asp-Lys-Asn-Thr-Phe-Ile-Tyr-Ser-Arg-

  41                                        50
  Pro-Glu-Pro-Val-Lys-Ala-Ile-Cys-Lys-Gly-

  51                                        60
  Ile-Ile-Ala-Ser-Lys-Asn-Val-Leu-Thr-Thr-

  61                                        70
  Ser-Glu-Phe-Tyr-Leu-Ser-Asp-Cys-Asn-Val-

  71                                        80
  Thr-Ser-Arg-Pro-Cys-Lys-Tyr-Lys-Leu-Lys-

  81                                        90
  Lys-Ser-Thr-Asn-Lys-Phe-Cys-Val-Thr-Cys-

  91                                       100
  Glu-Asn-Gln-Ala-Pro-Val-His-Phe-Val-Gly-

  101            104
  Val-Gly-Ser-Cys
```

und entweder (Z)-1-p-Dimethylaminoethoxyphenyl-1,2-diphenyl-1-buten, Citratsalz, oder 10-[3-(4-Methylpiperazin-1-yl)-propyl]-2-trifluormethylphenothiazin.

9. Protein mit der folgenden Aminosäuresequenz:

```
  1     2     3     4     5     6     7     8     9    10
<Glu-Asp-Trp-Leu-Thr-Phe-Gln-Lys-Lys-His-

 11                                            20
Ile-Thr-Asn-Thr-Arg-Asp-Val-Asp-Cys-Asp-

 21                                            30
Asn-Ile-Met-Ser-Thr-Asn-Leu-Phe-His-Cys-

 31                                            40
Lys-Asp-Lys-Asn-Thr-Phe-Ile-Tyr-Ser-Arg-

 41                                            50
Pro-Glu-Pro-Val-Lys-Ala-Ile-Cys-Lys-Gly-

 51                                            60
Ile-Ile-Ala-Ser-Lys-Asn-Val-Leu-Thr-Thr-

 61                                            70
Ser-Glu-Phe-Tyr-Leu-Ser-Asp-Cys-Asn-Val-

 71                                            80
Thr-Ser-Arg-Pro-Cys-Lys-Tyr-Lys-Leu-Lys-

 81                                            90
Lys-Ser-Thr-Asn-Lys-Phe-Cys-Val-Thr-Cys-

 91                                           100
Glu-Asn-Gln-Ala-Pro-Val-His-Phe-Val-Gly-

101           104
Val-Gly-Ser-Cys
```

## Revendications

1. Produit pharmaceutique bioactif comprenant
   une protéine ayant un poids moléculaire d'environ 12000 Daltons par spectrométrie de masse ou d'environ 14500 Daltons par électrophorèse sur gel, et une composition d'acides d'aminés approximativement comme suit:

| Reste d'acide aminé | % molaire (Hydrolyse acide 24 h.) |
|---|---|
| Acide aspartique/asparagine | 13,39 |
| Thréonine | 9,84 |
| Sérine | 8,08 |
| Acide glutamique/glutamine | 5,88 |
| Proline | 3,98 |
| Glycine | 2,98 |
| Alanine | 2,92 |
| Cystine/2 | 7,77 |
| Valine | 7,77 |
| Méthionine | 0,94 |
| Isoleucine | 5,29 |
| Leucine | 4,95 |
| Tyrosine | 2,85 |
| Phénylalaline | 5,73 |
| Histidine | 2,99 |
| Lysine | 11,78 |
| Arginine | 2,85 |
| Tryptophane | Non déterminé |
| Total approximatif | 99,99% |

et soit (Z)-1-p-diméthylaminoéthoxyphényl-1,2-diphényl-1-butène, sel citrate (tamoxifène, sel citrate), soit 10-[3-(4-methylpipérazin-1-yl )-propyl]-2-trifluoromethylphénothiazine (trifluopérazine).

2. Produit pharmaceutique bioactif comprenant une protéine ayant un poids moléculaire d'environ 12000 Daltons par spectrométrie de masse ou d'environ 14500 Daltons par électrophorèse sur gel, et une composition d'acides aminés approximativement comme suit:

| Acide aminé | Nombre de restes par molécule |
|---|---|
| Acide aspartique | 6 |
| Asparagine | 8 |
| Thréonine | 10 |
| Sérine | 8 |
| Acide glutamique | 3 |
| Acide pyroglutamique | 1 |
| Glutamine | 2 |
| Proline | 4 |
| Glycine | 3 |
| Alanine | 3 |
| Cystine/2 | 8 |
| Valine | 8 |
| Méthionine | 1 |
| Isoleucine | 6 |
| Leucine | 5 |
| Tyrosine | 3 |
| Phénylalaline | 6 |
| Histidine | 3 |
| Lysine | 12 |
| Arginine | 3 |
| Tryptophane | 1 |
| Total approximatif | 104 |

et soit (Z)-1-p-diméthylaminoéthoxyphényl-1,2-diphényl-1-butène, sel citrate, soit 10-[3-(4-méthylpipérazin-1-yl)-propyl]-2-trifluorométhylphénothiazine.

3. Produit pharmaceutique bioactif comprenant
une protéine pure pouvant être tirée d'oeufs fécondés, la protéine ayant un poids moléculaire d'environ 12000 Daltons par spectrométrie de masse, un point isoélectrique pI de 9,5 à 10,5 par focalisation isoélectrique, et un groupe terminal amino bloqué, la protéine étant essentiellement exempte d'hydrates de carbone, et
soit (Z)-1-p-diméthylaminoéthoxyphenyl-1,2-diphényl-1-butène, sel citrate, soit 10-[3-(4-méthylpipérazin-1-yl)-propyl]-2-trifluorométhylphénothiazine.

4. Combinaison selon la revendication 3, dans laquelle les acides aminés prédominant sont la lysine et la thréonine.

5. Combinaison selon la revendication 3, dans laquelle chaque molécule de la protéine contient exactement un reste de tryptophane.

6. Combinaison selon la revendication 3, dans laquelle chaque molécule de la protéine contient exactement un reste de méthionine.

7. Produit pharmaceutique bioactif comprenant
une protéine possédant une séquence d'acides aminés qui est au moins homologue à 60% à la séquence d'acides aminés qui suit:

```
    1    2    3    4    5    6    7    8    9   10
 <Glu-Asp-Trp-Leu-Thr-Phe-Gln-Lys-Lys-His-
   11                                       20
 Ile-Thr-Asn-Thr-Arg-Asp-Val-Asp-Cys-Asp-
   21                                       30
 Asn-Ile-Met-Ser-Thr-Asn-Leu-Phe-His-Cys-
   31                                       40
 Lys-Asp-Lys-Asn-Thr-Phe-Ile-Tyr-Ser-Arg-
   41                                       50
 Pro-Glu-Pro-Val-Lys-Ala-Ile-Cys-Lys-Gly-
   51                                       60
 Ile-Ile-Ala-Ser-Lys-Asn-Val-Leu-Thr-Thr-
   61                                       70
 Ser-Glu-Phe-Tyr-Leu-Ser-Asp-Cys-Asn-Val-
   71                                       80
 Thr-Ser-Arg-Pro-Cys-Lys-Tyr-Lys-Leu-Lys-
   81                                       90
 Lys-Ser-Thr-Asn-Lys-Phe-Cys-Val-Thr-Cys-
   91                                      100
 Glu-Asn-Gln-Ala-Pro-Val-His-Phe-Val-Gly-
  101            104
 Val-Gly-Ser-Cys
```

et soit (Z)-1-p-diméthylaminoéthoxyphényl-1,2-diphényl-1-butène, sel citrate, soit 10-[3-(4-méthylpipérazin-1-yl)-propyl]-2-trifluorométhylphénothiazine.

8. Produit pharmaceutique bioactif comprenant
   une protéine possédant un fragment d'acides aminés qui a une longueur d'au moins 15 restes d'acides aminés, lequel fragment possède une séquence d'acides aminés qui correspond à un quelconque fragment de même longueur de la séquence d'acides aminés qui suit:

```
  1     2     3     4     5     6     7     8     9     10
Glu-Asp-Trp-Leu-Thr-Phe-Gln-Lys-Lys-His-
 11                                        20
Ile-Thr-Asn-Thr-Arg-Asp-Val-Asp-Cys-Asp-
 21                                        30
Asn-Ile-Met-Ser-Thr-Asn-Leu-Phe-His-Cys-
 31                                        40
Lys-Asp-Lys-Asn-Thr-Phe-Ile-Tyr-Ser-Arg-
 41                                        50
Pro-Glu-Pro-Val-Lys-Ala-Ile-Cys-Lys-Gly-
 51                                        60
Ile-Ile-Ala-Ser-Lys-Asn-Val-Leu-Thr-Thr-
 61                                        70
Ser-Glu-Phe-Tyr-Leu-Ser-Asp-Cys-Asn-Val-
 71                                        80
Thr-Ser-Arg-Pro-Cys-Lys-Tyr-Lys-Leu-Lys-
 81                                        90
Lys-Ser-Thr-Asn-Lys-Phe-Cys-Val-Thr-Cys-
 91                                       100
Glu-Asn-Gln-Ala-Pro-Val-His-Phe-Val-Gly-
101           104
Val-Gly-Ser-Cys
```

et soit (Z)-1-p-diméthylaminoéthoxyphényl-1,2-diphényl-1-butène, sel citrate, soit 10-[3-(4-méthylpipérazin-1-yl)-propyl]-2-trifluorométhylphénothiazine.

**9.** Protéine possédant la séquence d'acides aminés qui suit:

```
  1     2     3     4     5     6     7     8     9     10
<Glu-Asp-Trp-Leu-Thr-Phe-Gln-Lys-Lys-His-
 11                                        20
Ile-Thr-Asn-Thr-Arg-Asp-Val-Asp-Cys-Asp-
 21                                        30
Asn-Ile-Met-Ser-Thr-Asn-Leu-Phe-His-Cys-
 31                                        40
Lys-Asp-Lys-Asn-Thr-Phe-Ile-Tyr-Ser-Arg-
 41                                        50
Pro-Glu-Pro-Val-Lys-Ala-Ile-Cys-Lys-Gly-
 51                                        60
Ile-Ile-Ala-Ser-Lys-Asn-Val-Leu-Thr-Thr-
```

```
61                                    70
Ser-Glu-Phe-Tyr-Leu-Ser-Asp-Cys-Asn-Val-
71                                    80
Thr-Ser-Arg-Pro-Cys-Lys-Tyr-Lys-Leu-Lys-
81                                    90
Lys-Ser-Thr-Asn-Lys-Phe-Cys-Val-Thr-Cys-
91                                    100
Glu-Asn-Gln-Ala-Pro-Val-His-Phe-Val-Gly-
101           104
Val-Gly-Ser-Cys
```

## FIG. 1

```
┌─────────────────────────────────────┐
│          INDUCED OVULATION          │
└─────────────────────────────────────┘
                   │
                   ▼
┌─────────────────────────────────────┐
│         FERTILIZATION OF EGGS        │
└─────────────────────────────────────┘
                   │
                   ▼
┌─────────────────────────────────────┐
│  HOMOGENIZATION OF EGG/EMBRYO MIXTURE │
└─────────────────────────────────────┘
                   │
                   ▼
┌─────────────────────────────────────┐
│            CENTRIFUGATION            │
└─────────────────────────────────────┘
                   │
                   ▼
┌─────────────────────────────────────┐
│              FILTRATION              │
└─────────────────────────────────────┘
                   │
                   ▼
┌─────────────────────────────────────┐
│               BIOASSAY               │
└─────────────────────────────────────┘
                   │
                   ▼
┌─────────────────────────────────────┐
│   FIRST ION-EXCHANGE CHROMATOGRAPHY   │
└─────────────────────────────────────┘
                   │
                   ▼
┌─────────────────────────────────────┐
│  SECOND ION-EXCHANGE CHROMATOGRAPHY   │
└─────────────────────────────────────┘
                   │
                   ▼
┌─────────────────────────────────────┐
│    SIZE-EXCLUSION CHROMATOGRAPHY     │
└─────────────────────────────────────┘
                   │
                   ▼
┌─────────────────────────────────────┐
│          STERILE FILTRATION          │
└─────────────────────────────────────┘
                   │
                   ▼
┌─────────────────────────────────────┐
│            LYOPHILIZATION            │
└─────────────────────────────────────┘
```

# FIG. 2

```
  1   2   3   4   5   6   7   8   9   10
<Glu-Asp-Trp-Leu-Thr-Phe-Gln-Lys-Lys-His-

 11                                      20
Ile-Thr-Asn-Thr-Arg-Asp-Val-Asp-Cys-Asp-

 21                                      30
Asn-Ile-Met-Ser-Thr-Asn-Leu-Phe-His-Cys-

 31                                      40
Lys-Asp-Lys-Asn-Thr-Phe-Ile-Tyr-Ser-Arg-

 41                                      50
Pro-Glu-Pro-Val-Lys-Ala-Ile-Cys-Lys-Gly-

 51                                      60
Ile-Ile-Ala-Ser-Lys-Asn-Val-Leu-Thr-Thr-

 61                                      70
Ser-Glu-Phe-Tyr-Leu-Ser-Asp-Cys-Asn-Val-

 71                                      80
Thr-Ser-Arg-Pro-Cys-Lys-Tyr-Lys-Leu-Lys-

 81                                      90
Lys-Ser-Thr-Asn-Lys-Phe-Cys-Val-Thr-Cys-

 91                                     100
Glu-Asn-Gln-Ala-Pro-Val-His-Phe-Val-Gly-

101         104
Val-Gly-Ser-Cys
```

## FIG. 3

| ONCONASE Dose (μg/ml) | 0.05 | 0.5 | 5.0 | 10.0 | 20.0 | 0 (STELAZINE ALONE) | $ED_{50}$ |
|---|---|---|---|---|---|---|---|
| ONCONASE Alone | 0 | 0 | 9.0 | 34.6 | 53.4 | – | 18.720 |
| ONCONASE + 2.5 μM STELAZINE | 0 | 0 | 14.7 | 39.4 | 60.4 | 0 | 14.490 |
| ONCONASE + 5 μM STELAZINE | 1.1 | 2.3 | 24.0 | 50.2 | 70.2 | 0 | 12.330 |
| ONCONASE + 10 μM STELAZINE | 90.8 | 90.8 | 92.3 | 93.6 | 93.8 | 86.3 | 0.027 |
| $ED_{50}$ – STELAZINE | 5.500 | 5.500 | 4.980 | 2.530 | 1.130 | 5.790 | – |

## FIG. 4

| ONCONASE Dose (μg/ml) | 0.05 | 0.5 | 5.0 | 10.0 | 20.0 | 0 (TAMOXIFEN ALONE) | $ED_{50}$ |
|---|---|---|---|---|---|---|---|
| ONCONASE Alone | 8.1 | 11.8 | 34.8 | 52.0 | 77.9 | – | 6.818 |
| ONCONASE + 5 μM TAMOXIFEN | 30.1 | 48.8 | 79.5 | 86.5 | 94.2 | 13.3 | 0.337 |
| ONCONASE + 10 μM TAMOXIFEN | 50.0 | 85.0 | 96.9 | 96.8 | 97.6 | 30.4 | 0.035 |
| ONCONASE + 15 μM TAMOXIFEN | 96.0 | 97.9 | 96.8 | 98.6 | 99.0 | 68.7 | 0.026 |
| $ED_{50}$ – TAMOXIFEN | 5.734 | 3.625 | 1.876 | 0.891 | 0.682 | 14.751 | – |

EP 0 500 589 B1